# EUROPEAN PATENT SPECIFICATION

(11) **EP 4 464 771 B1**
(45) Date of publication and mention of the grant of the patent: **24.06.2026**
(21) Application number: 23770177.6
(22) Date of filing: 09.02.2023
(51) Int. Cl.: C12M 1/12, C12M 3/00, A61L 9/14

(54) **INCUBATION DEVICE AND INCUBATION SYSTEM**
INKUBATIONSVORRICHTUNG UND INKUBATIONSSYSTEM
DISPOSITIF D'INCUBATION ET SYSTÈME D'INCUBATION

(30) Priority: 17.03.2022 JP 2022042686
(43) Date of publication of application: 20.11.2024
(73) Proprietor: PHC Holdings Corporation, Tokyo 100-8403 (JP)
(72) Inventor: SATO, Masakazu, Toon-shi, Ehime 791-0395 (JP); YOSHIDA, Kaori, Toon-shi, Ehime 791-0395 (JP); HITOMI, Tsugumasa, Toon-shi, Ehime 791-0395 (JP); OHTA, Akihiro, Toon-shi, Ehime 791-0395 (JP)
(74) Representative: Grünecker Patent- und Rechtsanwälte PartG mbB
(86) International application number: PCT/JP2023/004300
(87) International publication number: WO 2023/176216

(56) References cited:
- WO-A1-2017/169850
- WO-A1-2021/220784
- JP-A- 2011 160 672
- JP-A- 2020 092 715
- JP-A- H11 504 210
- US-A1- 2014 120 610
- US-A1- 2015 374 868
- US-A1- 2021 371 798
- アプリケーションノート, 過酸化水素蒸気による除染用途での湿度計測, VAISALA. 2021, pp. 1-4, [online], Internet: <https://www.vaisala.com/sites/default/files/documents/Humidity-measurements-and-hydrogen-peroxide-B212110JA.pdf>, [retrieval date 03 April 2023 (2023-04-03)] XP093089733

## Description

### Technical Field

The present disclosure relates to a culturing apparatus as defined in claim 1.

### Background Art

A culturing apparatus (incubator) for culturing a culture such as a cell or a microorganism is known in the related art (see, for example, Patent Literatures (hereinafter, each referred to as PTL) 1 and 2).

In the upper side, lower side, front side, and back side of the culture chamber in the culturing apparatus described in PTL 1, upper space, lower space, front space, and back space are respectively provided. A circulation fan that causes the air in the back space to flow in a horizontal direction in the culture chamber is disposed in the back space. A humidity sensor that detects the humidity in the culture chamber is disposed on the right side surface wall portion of the housing.

The culturing apparatus described in PTL 2 includes a gas generator that sterilizes the inside of a culture chamber by circulating hydrogen peroxide gas into the culture chamber. US 2014/120610 A1 and US 2021/371798 A1 each disclose culturing apparatus similar to the preamble of claim 1.

### Citation List

### Patent Literature

PTL 1
   Japanese Patent Application Laid-Open No. 2017-77208
PTL 2
   Japanese Patent Application Laid-Open No. 2010-17151

### Summary of Invention

### Technical Problem

In the culturing apparatus described in PTL 1, it is necessary to keep the culture chamber in a sterile state periodically. Accordingly, it is conceivable to decontaminate the culture chamber by using the gas generator described in PTL 2. When the humidity sensor is exposed to hydrogen peroxide gas, however, there is a possibility that the function thereof is reduced or a failure of the sensor occurs. In order to prevent such a problem, it is conceivable to decontaminate the culture chamber after removing the humidity sensor from the culture chamber, but in this case, there is a possibility that a humidity sensor contaminated outside the culture chamber is attached to the decontaminated culture chamber, and there is a possibility that a failure due to loss or dropping of the sensor caused by such removal occurs. Further, it is conceivable to decontaminate the culture chamber with a low-concentration hydrogen peroxide gas that does not adversely affect the humidity sensor, but in this case, there is a possibility that the decontamination may not satisfy the standard or a long decontamination time may be required in order to satisfy the standard.

An object of the present disclosure is to provide a culturing apparatus and a culture system capable of appropriately decontaminating a culture chamber without adversely affecting a humidity sensor.

### Solution to Problem

A culturing apparatus according to the present invention is defined in claim 1.

Preferred embodiments are set out in the dependent claims.

### Advantageous Effects of Invention

According to the culturing apparatus and the culture system of the present disclosure, it is possible to appropriately decontaminate a culture chamber without adversely affecting a humidity sensor.

### Brief Description of Drawings

FIG. 1 is a schematic longitudinal section of a culturing apparatus that constitutes a culture system as viewed from the right side;
FIG. 2 is a front view illustrating an outline of the inside of a culture chamber in a decontamination setup state in which a protection member is mounted on a plug connector;
FIG. 3 is a front view illustrating an outline of the inside of the culture chamber in a state where the protection member is mounted on a humidity sensor unit;
FIG. 4 is an exploded perspective view of the humidity sensor unit;
FIG. 5 is a longitudinal sectional view illustrating a state in which the protection member is mounted on the humidity sensor unit;
FIG. 6 is a front view illustrating the positional relationship between a magnet of the protection member and a protection sensor; and
FIG. 7 is a flowchart of an decontamination operation.

### Description of Embodiments

### [Embodiment]

Hereinafter, embodiments of the present disclosure will be described.

### <Configuration of Culture System>

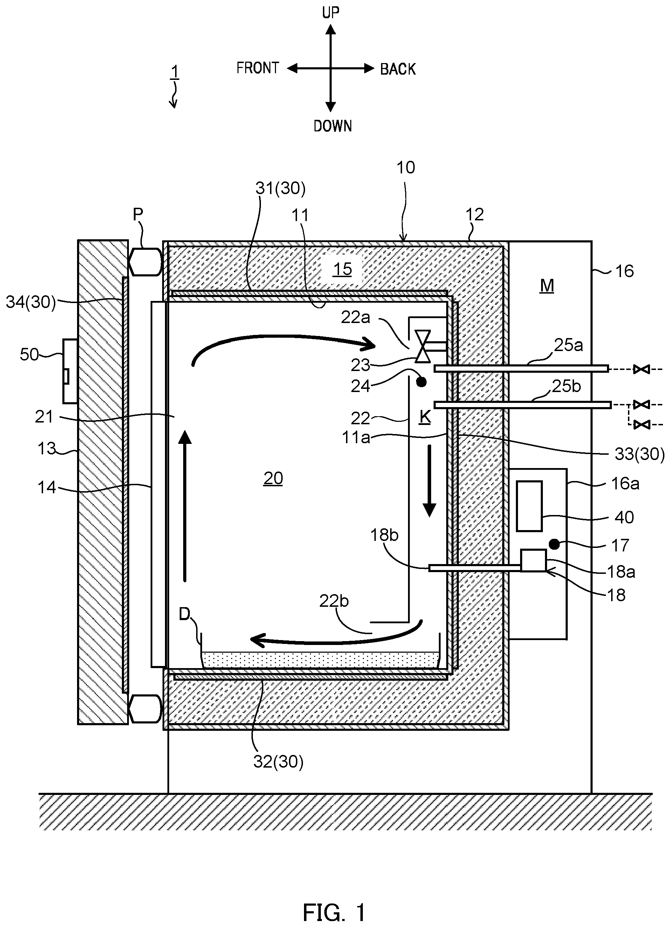

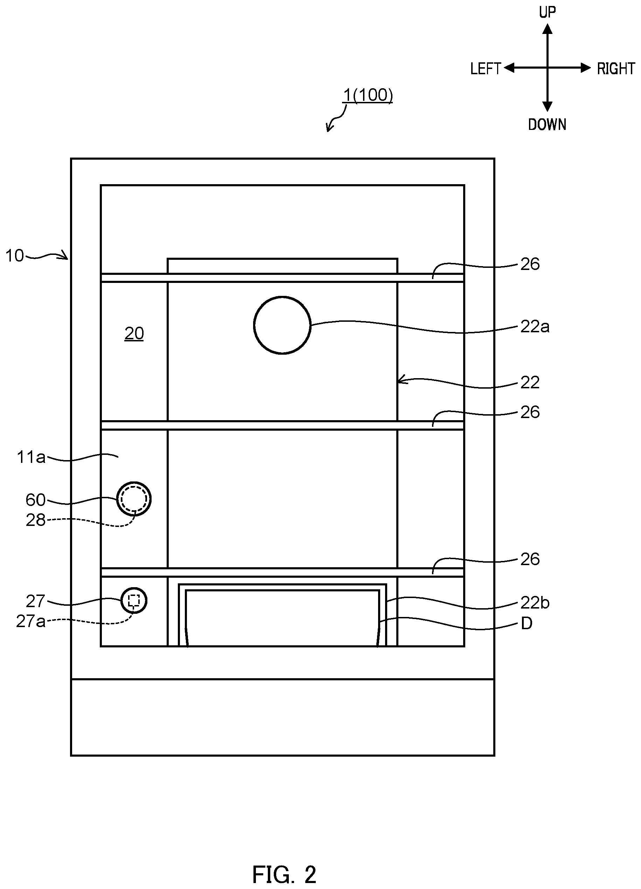

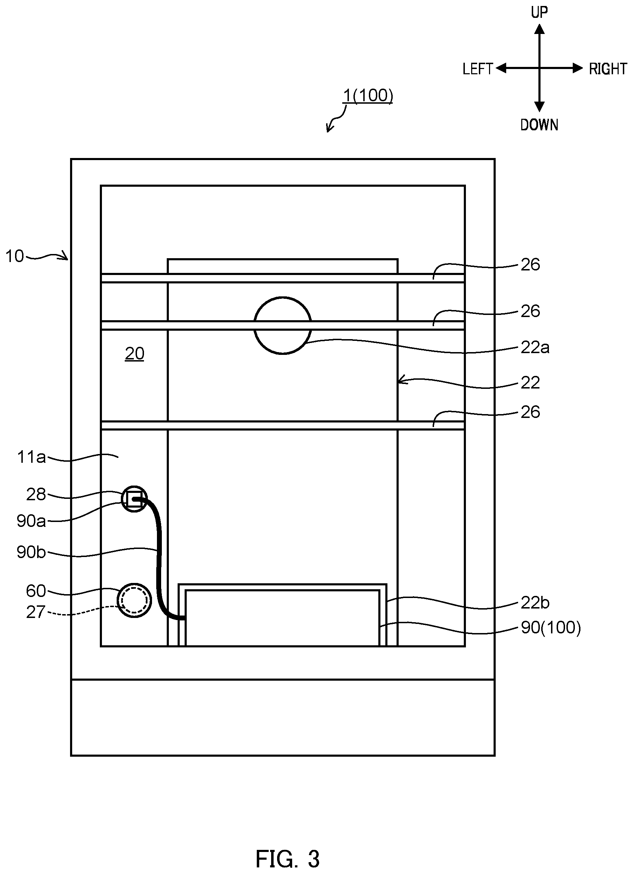

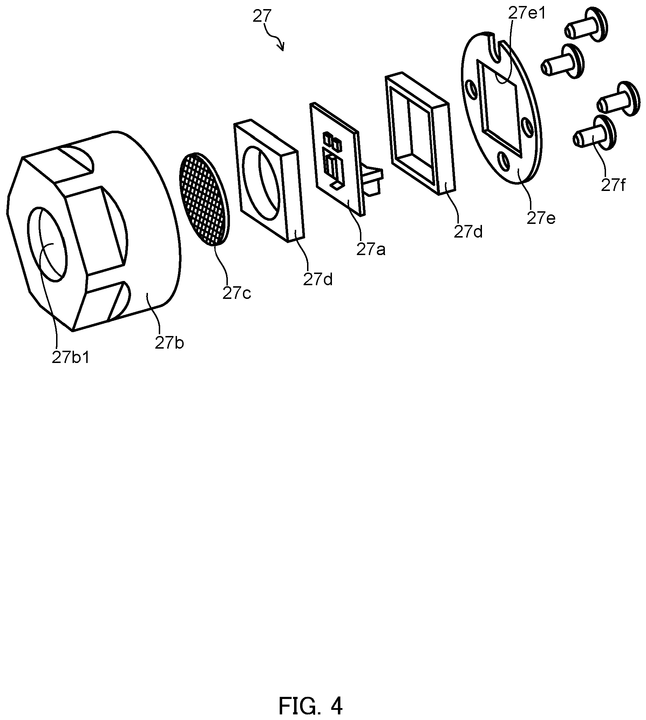

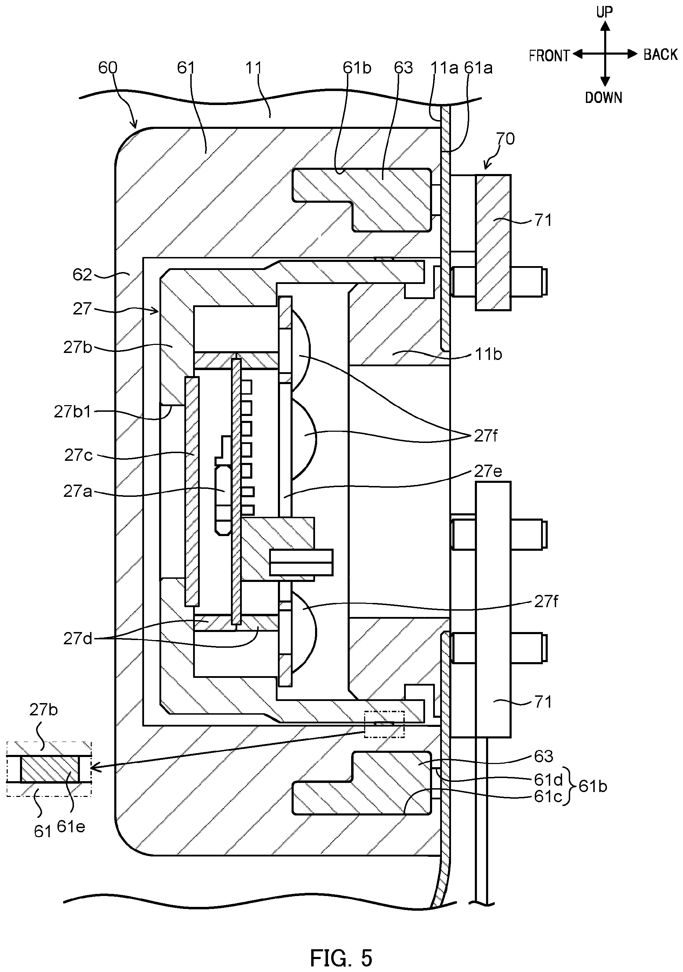

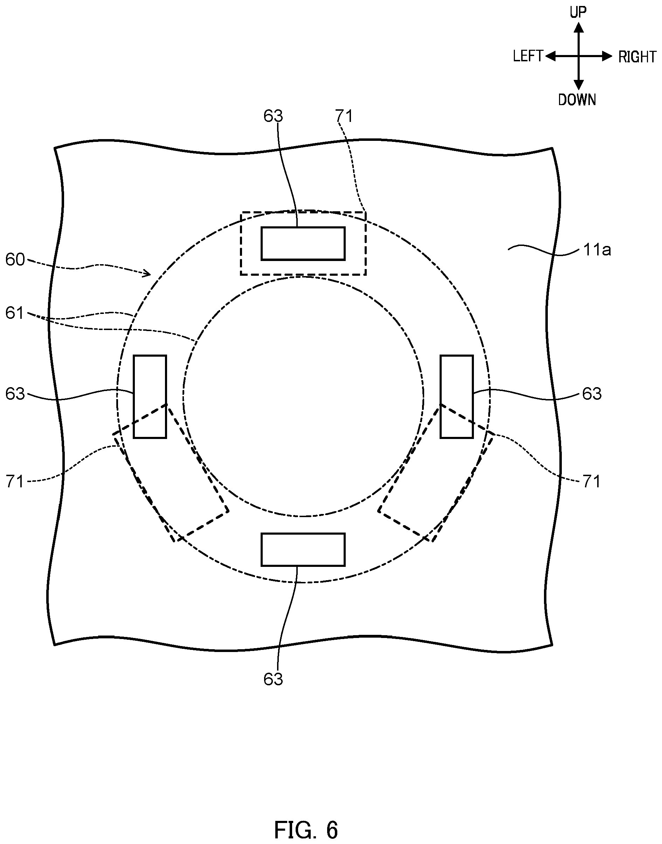

First, the configuration of a culture system will be described. FIG. 1 is a schematic longitudinal section of a culturing apparatus that constitutes a culture system as viewed from the right side. FIG. 2 is a front view illustrating an outline of the inside of a culture chamber in a decontamination setup state in which a protection member is mounted on a plug connector. FIG. 3 is a front view illustrating an outline of the inside of the culture chamber in a state where the protection member is mounted on a humidity sensor unit. FIG. 4 is an exploded perspective view of the humidity sensor unit. FIG. 5 is a longitudinal sectional view illustrating a state in which the protection member is mounted on the humidity sensor unit. FIG. 6 is a front view illustrating the positional relationship between a magnet of the protection member and a protection sensor. Hereinafter, a front side is a side in which a user of the culturing apparatus faces, and a back side is a side opposite to the front side. Further, the right side is a right side viewed from the user facing the culturing apparatus, and a left side is a side opposite to the right side. An upper side is an upper side in a case where the culturing apparatus is installed on a horizontal surface, and a lower side is a side opposite to the upper side.

Culturing apparatus 1 illustrated in FIG. 1 constitutes culture system 100 together with decontamination device 90 (see FIG. 3). Culturing apparatus 1 includes a box-shaped housing 10. Culture chamber 20 for culturing a culture such as a cell or a microorganism is formed inside housing 10. Housing 10 includes inner box 11, outer box 12, outer door 13, and inner door 14.

Inner box 11 is formed in a box shape including culture chamber 20 therein. Opening 21 of culture chamber 20 is formed on the front surface of inner box 11. Outer box 12 is formed in a box shape. Outer box 12 is disposed to cover portions of inner box 11 except for opening 21. Of inner box 11 and outer box 12, at least inner box 11 is formed of a nonmagnetic metal plate. The nonmagnetic metal may be exemplified by stainless steel. Heat insulating material 15 is disposed between inner box 11 and outer box 12.

Outer door 13 and inner door 14 open and close opening 21. Packing P is disposed at an outer edge of outer door 13. Operation unit 50 is disposed on an outer surface of outer door 13. Operation unit 50 accepts the input of various setting values for culture chamber 20. The various set values of culture chamber 20 are a set temperature, set humidity, a set concentration of O₂ gas, a set concentration of CO₂ gas, and the like. Operation unit 50 includes a display section that displays a state of culturing apparatus 1. The instruction for starting and stopping culturing apparatus 1 is performed by a power supply switch (not illustrated). Culturing apparatus 1 does not need to be provided with a function for controlling (setting) the O₂ gas concentration control (setting) function in culture chamber 20.

Heating unit 30 that heats culture chamber 20 is disposed in housing 10. Heating unit 30 includes top-surface heater 31 disposed on the upper side of inner box 11, bottom-surface heater 32 disposed on the lower side of inner box 11, rear-surface heater 33 disposed on the back side of inner box 11, side-surface heaters (not illustrated) disposed on the left and right sides of inner box 11, and outer door heater 34 disposed on outer door 13.

Duct 22 is disposed in culture chamber 20. Duct 22 is formed on back plate 11a that constitutes the rear surface of inner box 11 so as to extend vertically. Gas passage K is formed inside duct 22. In gas passage K, circulation fan 23 is disposed. By operating circulation fan 23, air in culture chamber 20 is sucked in from suction port 22a formed in an upper portion of duct 22, and this air is blown out to culture chamber 20 from blow-out port 22b provided in a lower portion of duct 22. As a result, forced circulation of the air as indicated with bold arrows is performed.

In duct 22, room temperature sensor 24 and gas supply devices 25a and 25b are disposed. Room temperature sensor 24 detects a temperature of culture chamber 20. Room temperature sensor 24 is disposed in a vicinity of suction port 22a and detects the temperature of air sucked in from suction port 22a. Gas supply devices 25a and 25b supply, to culture chamber 20, adjustment gas (for example, CO₂ gas, O₂ gas, and N₂ (nitrogen) gas) that adjust the O₂ gas concentration and the CO₂ gas concentration in culture chamber 20.

Humidification pan D for storing a liquid (specifically water) for humidification is installed between the lower portion of duct 22 and the bottom surface of inner box 11. Water stored in humidification pan D is sterilized by ultraviolet irradiation performed by a UV lamp (not illustrated).

The rear surface and the bottom surface of outer box 12 in housing 10 are covered with cover 16. A space between the rear surface of outer box 12 and cover 16 forms mechanical room M for disposing various types of equipment. Mechanical room M is provided with electrical box 16a. Electrical box 16a stores control device 40.

In addition, culturing apparatus 1 further includes outside air temperature sensor 17 and vapor supply device 18. Outside air temperature sensor 17 detects an ambient temperature of culturing apparatus 1. Vapor supply device 18 supplies vapor to culture chamber 20. Vapor supply device 18 includes vapor generator 18a and vapor supplier 18b.

The vapor generator 18a is disposed in electrical box 16a. Vapor generator 18a includes a heater (not illustrated). Vapor generator 18a is supplied with water from a tank (not illustrated), in which water is stored, by a pump (not illustrated), and generates vapor by evaporating the water with the heater. Vapor supplier 18b has a tubular shape, and supplies the vapor generated by vapor generator 18a to culture chamber 20.

As illustrated in FIGS. 2 and 3, culture chamber 20 is configured in such a way that at least one shelf 26 (not illustrated) can be detachably installed therein at a predetermined position in the vertical direction. Shelf 26 is supported by supports (not illustrated) disposed on the both left and right side surfaces of inner box 11 so as to be aligned in the vertical direction. Humidity sensor unit 27 and plug connector 28 are disposed on the rear surface of culture chamber 20. Protection member 60 is selectively mounted on humidity sensor unit 27 or plug connector 28 to cover the components.

Humidity sensor 27a that detects the humidity in culture chamber 20 is disposed inside humidity sensor unit 27. Humidity sensor unit 27 is disposed outside duct 22 on back plate 11a. By disposing humidity sensor unit 27 in such a manner, protection member 60 can be easily attached to and detached from humidity sensor unit 27 can be easily performed compared to a configuration in which humidity sensor unit 27 is disposed inside duct 22. Humidity sensor unit 27 is disposed on the left side of blow-out port 22b of duct 22. By disposing humidity sensor unit 27 in such a manner, the humidity of the air in culture chamber 20 before coming into contact with the culture can be detected by humidity sensor 27a, and the humidity control based on the detection result of humidity sensor 27a can be appropriately performed. Humidity sensor unit 27 is disposed at a position lower than the position of shelf 26 that is installed at the lowest position. By disposing humidity sensor unit 27 as described above, it is possible to surely prevent shelf 26 from being installed in front of humidity sensor unit 27, and protection member 60 can be easily attached to and detached from humidity sensor unit 27.

As illustrated in FIG. 3, power supply plug 90a of decontamination device 90 that decontaminates culture chamber 20 is connected to plug connector 28. Plug connector 28 is formed in a cylindrical shape having a diameter substantially the same as the outer diameter of holder 27b of humidity sensor unit 27.

Decontamination device 90 is installed between the lower portion of duct 22 and the bottom surface of inner box 11 in place of humidification pan D. Decontamination device 90 is driven by the power supplied through plug connector 28, power supply plug 90a, and cord 90b to generate hydrogen peroxide gas. The hydrogen peroxide gas generated in decontamination device 90 circulates between culture chamber 20 and duct 22 as indicated with bold arrows in FIG. 1, accompanying the activation of circulation fan 23. Circulation of the hydrogen peroxide gas decontaminates the inside of culture chamber 20.

Next, a detailed configuration of humidity sensor unit 27 will be described. As illustrated in FIG. 4, humidity sensor unit 27 includes humidity sensor 27a, holder 27b, filter 27c, a pair of packings 27d, and pressing plate 27e.

Humidity sensor 27a is constituted by an electrostatic capacitance type humidity sensor. Holder 27b is formed in a substantially cylindrical shape with one end surface closed and the other end surface open. Through-hole 27b1 is formed on one end surface of holder 27b. Inside holder 27b, filter 27c is disposed so as to block through-hole 27b1. Humidity sensor 27a located between the pair of packings 27d is disposed on the back side of filter 27c (the side opposite to through-hole 27b1). Pressing plate 27e is fixed to holder 27b by screw 27f on the back side of the rear packing 27d. Humidity sensor 27a and control device 40 are electrically connected to each other by a wire (not illustrated) that passes through opening 27e1 of pressing plate 27e. Humidity sensor unit 27 is attached to culture chamber 20 by screwing a portion on the other end surface side of holder 27b to the attacher 11b (see FIG. 5) that protrudes from back plate 11a of inner box 11.

Next, a detailed configuration of protection member 60 will be described. Protection member 60 illustrated in FIGS. 5 and 6 is formed of, for example, a resin or rubber having corrosion resistance to hydrogen peroxide gas, and is configured to be elastically deformable. Protection member 60 in the present embodiment is made of silicon rubber. Protection member 60 includes cylindrical base 61, and closer 62 that closes one end surface of base 61. As illustrated in FIGS. 3, 5, and 6, protection member 60 is mounted on humidity sensor unit 27 so as to prevent humidity sensor 27a from being exposed to culture chamber 20 by covering the outer periphery of holder 27b with base 61 and covering the front surface of holder 27b with closer 62. As illustrated in FIG. 2, protection member 60 is mounted on plug connector 28 so as to preventing plug connector 28 from being exposed to culture chamber 20 by covering the outer periphery of plug connector 28 with base 61 and covering the front surface of plug connector 28 with closer 62. That is, protection member 60 is selectively mounted on humidity sensor unit 27 or plug connector 28.

Base 61 is formed in a shape such that the entire surface of the other end surface 61a comes into contact with back plate 11a of inner box 11 when protection member 60 is mounted on humidity sensor unit 27 or plug connector 28. Further, the inner peripheral surface of the base 61 is configured to be in close contact with the outer peripheral surface of holder 27b and the outer peripheral surface of plug connector 28. Such a shape of base 61 makes it possible to prevent the hydrogen peroxide gas from coming into contact with humidity sensor unit 27 or plug connector 28.

Four magnet disposition grooves 61b, in each of which magnet 63 is disposed, are formed in the other end surface 61a of base 61. Four magnet disposition grooves 61b are formed at equal intervals along the outer periphery of base 61. That is, magnets 63 are intermittently disposed on base 61 so as to surround the periphery of humidity sensor unit 27 when viewed from the back side of the magnets in the mounting direction of protection member 60. Each magnet disposition groove 61b includes storage 61c in which magnet 63 is stored, and inserter 61d for inserting magnet 63 into storage 61c. Inserter 61d is formed in a shape smaller than the end portion of storage 61c on the inserter 61d side. The shape of such inserter 61d makes it possible to prevent magnet 63 stored in storage 61c from slipping out from protection member 60.

The inner diameter of base 61 is set to be substantially the same as the outer diameter of holder 27b of humidity sensor unit 27 and the diameter of plug connector 28. As illustrated in a partially enlarged view of FIG. 5 indicated by a one-dot chain line, a protrusion 61e that protrudes in a shape of a line along the inner peripheral direction of base 61 is formed on the inner peripheral surface of base 61. Protrusion 61e is formed such that a diameter of the circle drawn by the leading end of protrusion 61e is smaller than the outer diameter of holder 27b and the diameter of plug connector 28. With such a shape of protrusion 61e, it is possible to mount protection member 60 on humidity sensor unit 27 or plug connector 28 with a state where protrusion 61e elastically deformed is in contact with humidity sensor unit 27 or plug connector 28. Therefore, even when a scratch is made on a part of other end surface 61a of base 61 and thus a gap is formed between other end surface 61a and back plate 11a of inner box 11, it is possible to prevent the hydrogen peroxide gas from coming into contact with a portion on the front surface side of humidity sensor unit 27 or plug connector 28.

Culturing apparatus 1 further includes protection sensor 70. Protection sensor 70 detects that protection member 60 is mounted on humidity sensor unit 27. Protection sensor 70 includes a plurality of reed switches 71, three reed switches 71 in the present embodiment.

Each reed switch 71 is electrically connected to control device 40. Each reed switch 71 is configured to open when no magnetic force is applied, and to close when a magnetic force is applied. Each reed switch 71 is disposed on the back side of back plate 11a of inner box 11, that is, reed switch 71 is disposed in such a way that back plate 11a is located between protection member 60 mounted on humidity sensor unit 27 and reed switch 71. When three reed switches 71 are viewed from the back side thereof in the mounting direction of protection member 60, the three reed switches 71 are disposed at equal intervals so as to surround the periphery of humidity sensor unit 27. That is, in the present embodiment, whether protection member 60 is mounted on humidity sensor unit 27 is detected by four magnets 63 and three reed switches 71. By disposing the different numbers of magnets 63 (at equal intervals) and reed switches 71 (at equal intervals) as described above, it is possible to keep the angle formed by a straight line connecting the center of protection member 60 and magnet 63 and a straight line connecting the center of humidity sensor unit 27 and reed switch 71 small, in the combination of magnet 63 and reed switch 71, which are closest to each other. In other words, it is possible to bring the distance between magnet 63 and reed switch 71 close to each other. Specifically, in a configuration in which four magnets 63 are disposed at equal intervals and four reed switches 71 are disposed at equal intervals, the angle described above becomes 22.5 degrees or less, whereas, in a configuration in which four magnets 63 are disposed at equal intervals and three reed switches 71 are disposed at equal intervals as in the present embodiment, the angle described above can be kept to be 15 degrees or less. Due to the disposition of the three reed switches 71 as described above, regardless of the positions of the four magnets 63 when protection member 60 is mounted on humidity sensor unit 27, at least one reed switch 71 is close to at least one magnet 63, and is closed by the magnetic force of magnet 63. That is, regardless of the positions of the four magnets 63 when protection member 60 is mounted on humidity sensor unit 27, protection sensor 70 can detect that protection member 60 is mounted on humidity sensor unit 27.

The magnetic force of magnet 63 is preferably set such that when at least the other end surface 61a of base 61 is in contact with back plate 11a, reed switch 71 is closed, and when the other end surface 61a of base 61 is not in contact with back plate 11a, reed switch 71 is opened. In the present embodiment, protrusion 61e is formed on base 61 of protection member 60. Therefore, the magnetic force of magnet 63 is set such that when the distance from other end surface 61a of base 61 to back plate 11a is less than an allowable distance, reed switch 71 is closed, and when the distance from other end surface 61a of base 61 to back plate 11a is equal to or more than the allowable distance, reed switch 71 is opened. The allowable distance is the maximum distance at which protrusion 61e as a whole can be brought into close contact with the entire outer periphery of holder 27b, and is approximately 5 mm in the present embodiment. Thus, it is possible to improve usability by decontaminating while preventing the hydrogen peroxide gas from coming into contact with humidity sensor unit 27 or plug connector 28 even when the other end surface 61a is not in contact with back plate 11a.

### <Operation of Culture System>

Next, the operation of culture system 100 will be described.

### <Culture Operation>

First, the culture operation of culturing a culture will be described as an operation of culture system 100. Before starting the culture operation, the user mounts protection member 60 on plug connector 28. By mounting protection member 60 on plug connector 28 in this manner, it is possible to prevent the loss of protection member 60 and to prevent moisture generated by humidification in culture chamber 20 from adhering to plug connector 28. Further, humidity sensor 27a of humidity sensor unit 27, to which protection member 60 is not attached, can detect the humidity in culture chamber 20. The user installs humidification pan D in culture chamber 20 and places, for example, a Petri dish into which a culture has been put, on shelf 26.

When information on starting the culture operation is input into operation unit 50 by the user, control device 40 controls circulation fan 23, gas supply devices 25a and 25b, and heating unit 30 such that the concentrations and temperatures of the gases become suitable for culturing the culture in housing 10 (culture chamber 20). When bottom-surface heater 32 generates heat, water stored in humidification pan D evaporates, and culture chamber 20 is humidified so that the humidity in culture chamber 20 reaches the target value. Control device 40 selects one of the normal humidification control and the rapid humidification control based on information input to operation unit 50 by the user during the culture operation, and causes culture chamber 20 to be humidified such that the humidity detected by humidity sensor unit 27 becomes a target value. The normal humidification control is control in which culture chamber 20 is humidified by evaporating the water in humidification pan D with heat generation of bottom-surface heater 32 without operating vapor supply device 18. The rapid humidification control is control in which culture chamber 20 is humidified by evaporating the water in humidification pan D with the heat generation of bottom-surface heater 32 and by operating vapor supply device 18, and therefore, culture chamber 20 can be humidified quickly as compared with the normal humidification control. Details of the normal humidification control and the rapid humidification control will be omitted.

### <Decontamination Operation>

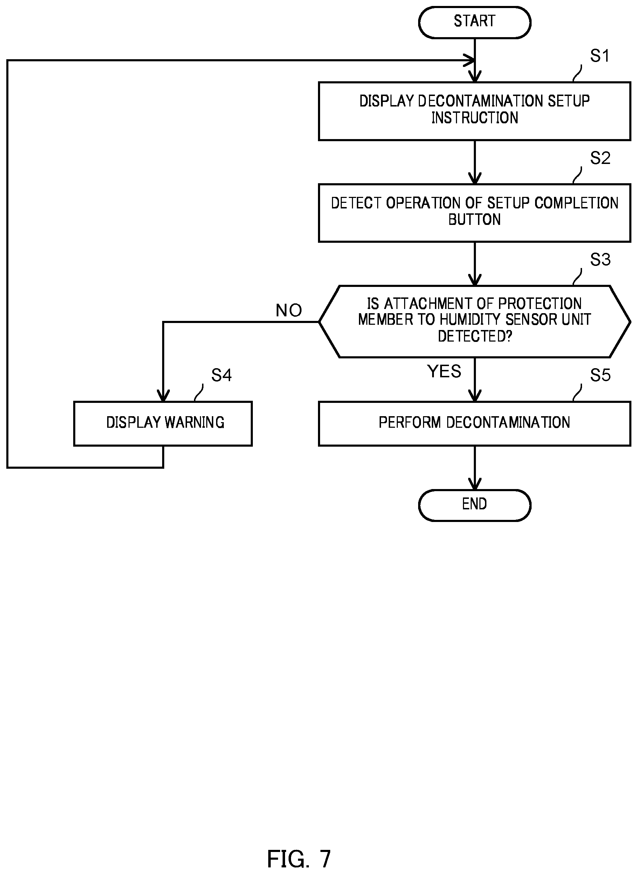

Next, culture system 100 will be described as the decontamination operation with reference to FIG. 7. The decontamination operation is performed to sterilize the culture that has been cultured by the culture operation performed earlier or eliminate microorganisms or the like due to the culture. FIG. 7 is a flowchart of the decontamination operation.

When information indicating that the decontamination operation is started by the user is input into operation unit 50, control device 40 causes operation unit 50 to display a decontamination setup instruction as illustrated in FIG. 7 (step S1). The setup instruction includes an instruction to specify the installation position of shelf 26, an instruction to mount protection member 60 on humidity sensor unit 27, and an instruction to connect the power supply to decontamination device 90.

In a case where at least one shelf 26 is installed below plug connector 28 or at the same height as plug connector 28 when the user opens outer door 13 and inner door 14, the user moves shelf 26 such that all shelves 26 are located above plug connector 28. Next, the user removes protection member 60 from plug connector 28 and mounts protection member 60 on humidity sensor unit 27 based on the setup instruction. In a case where protection member 60 is mounted such that a distance from other end surface 61a of protection member 60 to back plate 11a is less than the allowable distance, at least one reed switch 71 transitions from the open state to the closed state due to the magnetic force of magnet 63 of protection member 60. In a case where protection member 60 is not mounted such that the distance between the other end surface 61a of protection member 60 and back plate 11a is less than the allowable distance, the state in which all of the reed switches 71 are open is maintained.

Next, the user stands humidification pan D against the wall surface of culture chamber 20, installs decontamination device 90 (not illustrated), and connects power supply plug 90a to plug connector 28. Subsequently, the user closes the outer door 13 and the inner door 14, and presses the setup completion button. When operation unit 50 is configured by a touch panel, the setup completion button is a button displayed on the touch panel, and when operation unit 50 is not configured by a touch panel, the setup completion button is a physical button.

When the operation of the setup completion button is detected (step S2), control device 40 determines whether protection member 60 is mounted on humidity sensor unit 27, based on the detection result in reed switch 71 (step S3). In a case where all reed switches 71 are open and the mounting of protection member 60 on humidity sensor unit 27 cannot be detected (step S3: NO), control device 40 causes operation unit 50 to display a warning indicating that protection member 60 is not mounted on humidity sensor unit 27 without performing decontamination by decontamination device 90 (step S4). Subsequently, control device 40 performs the processing in step S1.

In a case where at least one reed switch 71 is closed and the mounting of humidity sensor unit 27 on protection member 60 can be detected (step S3: YES), control device 40 performs decontamination by decontamination device 90 (step S5). In the processing of step S5, for example, control device 40 may start the decontamination when control device 40 detects the operation of a decontamination start button (constituted by a button displayed on the touch panel or a physical button) after the detection of the mounting of protection member 60 on humidity sensor unit 27. Alternatively, control device 40 may start decontamination immediately when control device 40 detects that protection member 60 is mounted on humidity sensor unit 27. When decontamination is performed, control device 40 supplies power to decontamination device 90 to drive the decontamination device, thereby generating hydrogen peroxide gas, and drives circulation fan 23 to circulate the hydrogen peroxide gas. Circulation of the hydrogen peroxide gas decontaminates the inner wall of culture chamber 20 and shelf 26. When a predetermined time has elapsed since the decontamination was started, control device 40 stops circulation fan 23 and decontamination device 90 to end the decontamination. After the decontamination ends, decontamination device 90 is removed, shelf 26 and humidification pan D, which have been moved, are returned to their original positions, protection member 60 is removed from humidity sensor unit 27 and is mounted on plug connector 28. When control device 40 detects the mounting of protection member 60 on humidity sensor unit 27 in the culture operation, control device 40 may cause operation unit 50 to display a warning to prompt the user to remove protection member 60 from humidity sensor unit 27 and mount protection member 60 to plug connector 28.

As described above, when control device 40 can detect the mounting of protection member 60 on humidity sensor unit 27, control device 40 causes decontamination device 90 to perform decontamination, whereas when control device 40 cannot detect the mounting of protection member 60 on humidity sensor unit 27, control device 40 does not cause decontamination device 90 to perform decontamination. Therefore, it is possible to prevent humidity sensor unit 27 from being corroded by the hydrogen peroxide gas, and to decontaminate culture chamber 20 appropriately without adversely affecting humidity sensor unit 27. Further, there is no need to take humidity sensor unit 27 out of culture chamber 20 in order to prevent humidity sensor unit 27 from being corroded. Accordingly, it is possible to prevent culture chamber 20 from being contaminated when humidity sensor unit 27, (which was contaminated outside culture chamber 20) is attached to the decontaminated culture chamber 20. Further, the time required to attach and detach humidity sensor unit 27 is eliminated, and it is possible to shorten the time spent on decontamination. Further, it is not necessary to decrease the concentration of the hydrogen peroxide gas, and thus, it is possible to satisfy the standard relating to decontamination of culture chamber 20 without spending a long time.

Reed switch 71 (which is closed by a magnetic force) is applied as protection sensor 70 for detecting the mounting of protection member 60 on humidity sensor unit 27. Therefore, it is possible to detect the mounting of protection member 60 with a simple configuration without the need to form a window for allowing light to pass through back plate 11a, as in the case of applying a proximity sensor using infrared rays as protection sensor 70, for example.

It is needless to say that the present disclosure is not limited to the embodiments described thus far, and that various modifications can be made without departing from the scope of the present disclosure. The above-described embodiments and variations described below may be combined in any manner in an applicable range.

For example, a configuration in which protection member 60 is selectively mounted on humidity sensor unit 27 or plug connector 28 has been exemplified, but protection member 60 may be configured to be mountable on humidity sensor unit 27 while not being mountable on plug connector 28. Further, protection member 60 may have a shape that blocks only through-hole 27b1 of humidity sensor unit 27. In such a case, protection member 60 may be configured to be detachably mounted to humidity sensor unit 27, or may be attached to the leading end surface of holder 27b via a hinge so that through hole 27b1 can be opened and closed. Protection member 60 may be fixed to back plate 11a so as to cover humidity sensor unit 27 or plug connector 28. Further, protection member 60 does not need to include protrusion 61e. Further, base 61 and closer 62 may be formed of a material that does not undergo elastic deformation, while protrusion 61e may be formed of a material that undergoes elastic deformation.

Only one magnet 63 may be disposed in protection member 60, or a ring-shaped magnet that surrounds the periphery of humidity sensor unit 27 may be disposed. Protection sensor 70 may be constituted by only one reed switch 71. A window that transmits infrared rays may be formed in back plate 11a, and a proximity sensor that uses infrared rays may be applied as protection sensor 70.

Humidity sensor unit 27 may be disposed inside duct 22, may be disposed above blow-out port 22b outside duct 22, or may be disposed at a position above shelf 26 installed at the lowest position.

As decontamination device 90, a decontamination device that generates a gas with a decontamination effect other than hydrogen peroxide gas, such as peracetic acid gas, may be applied. Culturing apparatus 1 does not need to include vapor supply device 18 or to have a rapid humidification control function using vapor supply device 18.

The present disclosure can be applied to a culturing apparatus and a culture system.

### Reference Signs List

1 Culturing apparatus
10 Housing
11 Inner Box
11a Back plate
11b Attacher
12 Outer box
13 Outer Door
14 Inner Door
15 Heat insulating material
16 Cover
16a Electrical box
17 Outside air temperature sensor
18 Vapor supply device
18a Vapor generator
18b Vapor supplier
20 Culture chamber
21, 27e1 Opening
22 Duct
22a Suction port
22b Blow-out port
23 Circulation fan
24 Room temperature sensor
25a, 25b Gas supply device
26 Shelf
27 Humidity sensor unit
27a Humidity sensor
27b Holder
27b1 Through-hole
27c Filter
27d, P Packing
27e Pressing plate
27f Screw
28 Plug connector
30 Heating unit
31 Top-surface heater
32 Bottom-surface heater
33 Rear-surface heater
34 Outer door heater
40 Control device
50 Operation unit
60 Protection member
61 Base
61a Other end surface
61b Magnet disposition groove
61c: Storage
61d Inserter
61e Protrusion
62 Closer
63 Magnet
70 Protection sensor
71 Reed switch
90 Decontamination device
90a Power supply plug
90b Cord
100 Culture system
D Humidification pan
K Gas passage
M Mechanical room

## Claims

1. A culturing apparatus (1) comprising:
a housing (10) that includes a culture chamber (20);
a humidity sensor (27a) that detects humidity in the culture chamber (20);
a protection member (60) that prevents the humidity sensor (27a) from being exposed to the culture chamber (20); and
a control device (40) that controls a decontamination device (90) performing decontamination of the culture chamber (20),
**characterized by**
a protection sensor (70) disposed in the housing, (10), wherein
the protection member (60) is configured to be detachably mounted to the humidity sensor (27a) so as to prevent the humidity sensor (27a) from being exposed to the culture chamber (20),
the protection sensor (70) is configured to detect whether the protection member (60) is mounted to said humidity sensor (27a), and
the control device (40) is configured to cause the decontamination device (90) to perform the decontamination when the protection sensor (70) detects that the protection member (60) is mounted, and to not cause the decontamination device (90) to perform the decontamination when the protection sensor (70) does not detect that the protection member (60) is mounted.

2. The culturing apparatus (1) according to claim 1, wherein:
a magnet (63) is disposed in the protection member (60); and
the protection sensor (70) is configured to detect a magnetic force of the magnet (63) when the protection member (60) is mounted.

3. The culturing apparatus (1) according to claim 2, wherein
at least one of the protection sensor (70) and/or the magnet (63) is disposed continuously or intermittently such that the at least one of the protection sensor (70) and/or the magnet (63) surrounds a periphery of the humidity sensor (27a) when viewed from a back side in a mounting direction of the protection member (60).

4. The culturing apparatus (1) according to any one of claims 1 to 3, wherein:
a plug connector (28) to which a power supply plug (90a) of the decontamination device (90) is connected is disposed in the culture chamber (20), and
the protection member (60) is configured to be detachably mounted to the plug connector (28) so as to prevent the plug connector (28) from being exposed to the culture chamber (20), and is selectively mounted on the humidity sensor (27a) or the plug connector (28).

5. The culturing apparatus (1) according to any one of claims 1 to 4, further comprising:
a humidity adjustment device that adjusts the humidity in the culture chamber (20) by supplying vapor to the culture chamber (20), wherein
a duct (22) disposed in the culture chamber (20) and a fan (23) for circulating the vapor are disposed in the housing (10), the vapor being circulated between the duct (22) and the culture chamber (20), and
the humidity sensor (27a) is disposed outside the duct (22) in the culture chamber (20).

6. The culturing apparatus (1) according to claim 5, wherein
the humidity sensor (27a) is disposed in a position adjacent to an outlet for the vapor in the duct (22).

7. The culturing apparatus (1) according to claim 6, wherein:
the culture chamber (20) is configured in such a way that a shelf (26) on which a culture is placed is allowed to be installed at a predetermined position in a vertical direction; and
the humidity sensor (27a) is disposed below a lowest position at which the shelf is allowed to be installed.

## Patentansprüche

1. Eine Kultivierungsvorrichtung (1), umfassend:
ein Gehäuse (10), das eine Kulturkammer (20) umfasst;
einen Feuchtigkeitssensor (27a), der die Feuchtigkeit in der Kulturkammer (20) erfasst;
ein Schutzelement (60), das verhindert, dass der Feuchtigkeitssensor (27a) der Kulturkammer (20) ausgesetzt wird; und
eine Steuervorrichtung (40), die eine Dekontaminationsvorrichtung (90) steuert, die eine Dekontamination der Kulturkammer (20) durchführt,
**gekennzeichnet durch**
einen Schutzsensor (70) der im Gehäuse (10) angeordnet ist, wobei
das Schutzelement (60) konfiguriert ist, abnehmbar an dem Feuchtigkeitssensor (27a) angebracht werden zu können, um zu verhindern, dass der Feuchtigkeitssensor (27a) der Kulturkammer (20) ausgesetzt wird,
der Schutzsensor (70) konfiguriert ist, zu erfassen, ob das Schutzelement (60) an dem Feuchtigkeitssensor (27a) angebracht ist, und
die Steuervorrichtung (40) konfiguriert ist, die Dekontaminationsvorrichtung (90) zu veranlassen, die Dekontamination durchzuführen, wenn der Schutzsensor (70) erfasst, dass das Schutzelement (60) angebracht ist, und die Dekontaminationsvorrichtung (90) nicht veranlasst, die Dekontamination durchzuführen, wenn der Schutzsensor (70) nicht erfasst, dass das Schutzelement (60) angebracht ist.

2. Die Kultivierungsvorrichtung (1) gemäß Anspruch 1, wobei:
ein Magnet (63) in dem Schutzelement (60) angeordnet ist; und
der Schutzsensor (70) konfiguriert ist, eine Magnetkraft des Magneten (63) zu erfassen, wenn das Schutzelement (60) angebracht ist.

3. Die Kultivierungsvorrichtung (1) gemäß Anspruch 2, wobei
mindestens einer von dem Schutzsensor (70) und/oder dem Magnet (63) kontinuierlich oder intermittierend so angeordnet ist, dass der mindestens eine von dem Schutzsensor (70) und/oder dem Magnet (63), von einer Rückseite, in einer Befestigungsrichtung des Schutzelements (60) aus gesehen, einen Umfang des Feuchtigkeitssensors (27a) umgibt.

4. Die Kultivierungsvorrichtung (1) gemäß einem der Ansprüche 1 bis 3, wobei:
ein Steckverbinder (28), an den ein Stromversorgungsstecker (90a) der Dekontaminationsvorrichtung (90) angeschlossen ist, in der Kulturkammer (20) angeordnet ist, und
das Schutzelement (60) ausgebildet ist, abnehmbar an dem Steckverbinder (28) angebracht werden zu können, um zu verhindern, dass der Steckverbinder (28) der Kulturkammer (20) ausgesetzt wird, und wahlweise an dem Feuchtigkeitssensor (27a) oder dem Steckverbinder (28) angebracht ist.

5. Die Kultivierungsvorrichtung (1) gemäß einem der Ansprüche 1 bis 4, ferner umfassend:
eine Feuchteregulierungsvorrichtung, die die Feuchtigkeit in der Kulturkammer (20) durch Zuführen von Dampf in die Kulturkammer (20) reguliert, wobei
ein in der Kulturkammer (20) angeordneter Kanal (22) und ein Gebläse (23) zum Umwälzen des Dampfes im Gehäuse (10) angeordnet sind, wobei der Dampf zwischen dem Kanal (22) und der Kulturkammer (20) umgewälzt wird, und
der Feuchtigkeitssensor (27a) außerhalb des Kanals (22) in der Kulturkammer (20) angeordnet ist.

6. Die Kultivierungsvorrichtung (1) gemäß Anspruch 5, wobei
der Feuchtigkeitssensor (27a) an einer Stelle neben einem Auslass für den Dampf im Kanal (22) angeordnet ist.

7. Die Kultivierungsvorrichtung (1) gemäß Anspruch 6, wobei:
die Kulturkammer (20) ausgebildet ist, dass ein Regal (26), auf dem eine Kultur platziert wird, an einer vorbestimmten Position in vertikaler Richtung installiert werden kann; und
der Feuchtigkeitssensor (27a) unterhalb einer untersten Position angeordnet ist, an der das Regal angebracht werden kann.

## Revendications

1. Appareil de culture (1) comprenant :
un boîtier (10) qui inclut une chambre de culture (20) ;
un capteur d'humidité (27a) qui détecte l'humidité dans la chambre de culture (20) ;
un élément de protection (60) qui empêche le capteur d'humidité (27a) d'être exposé à la chambre de culture (20) ; et
un dispositif de contrôle (40) qui contrôle un dispositif de décontamination (90) mettant en œuvre la décontamination de la chambre de culture (20),
**caractérisé par**
un capteur de protection (70) disposé dans le boîtier (10), dans lequel
l'élément de protection (60) est configuré pour être monté de manière détachable sur le capteur d'humidité (27a) afin d'empêcher que le capteur d'humidité (27a) soit exposé à la chambre de culture (20),
le capteur de protection (70) est configuré pour détecter si l'élément de protection (60) est monté ou non sur ledit capteur d'humidité (27a), et
le dispositif de contrôle (40) est configuré pour commander au dispositif de décontamination (90) de mettre en œuvre la décontamination quand le capteur de protection (70) détecte que l'élément de protection (60) est monté, et pour ne pas commander au dispositif de décontamination (90) de mettre en œuvre la décontamination quand le capteur de protection (70) ne détecte pas que l'élément de protection (60) est monté.

2. Appareil de culture (1) selon la revendication 1, dans lequel :
un aimant (63) est disposé dans l'élément de protection (60) ; et
le capteur de protection (70) est configuré pour détecter une force magnétique de l'aimant (63) quand l'élément de protection (60) est monté.

3. Appareil de culture (1) selon la revendication 2, dans lequel
au moins un élément parmi le capteur de protection (70) et l'aimant (63) est disposé de manière continue ou intermittente de sorte que ledit au moins un élément parmi le capteur de protection (70) et l'aimant (63) entoure une périphérie du capteur d'humidité (27a) vu depuis un côté arrière en direction de montage de l'élément de protection (60).

4. Appareil de culture (1) selon l'une quelconque des revendications 1 à 3, dans lequel :
un connecteur de prise (28) auquel est connectée une prise d'alimentation (90a) du dispositif de décontamination (90) est disposé dans la chambre de culture (20), et
l'élément de protection (60) est configuré pour être monté de manière détachable sur le connecteur de prise (28) de manière à empêcher que le connecteur de prise (28) soit exposé à la chambre de culture (20), et est monté sélectivement sur le capteur d'humidité (27a) ou sur le connecteur de prise (28).

5. Appareil de culture (1) selon l'une quelconque des revendications 1 à 4, comprenant en outre :
un dispositif de régulation d'humidité qui régule l'humidité dans la chambre de culture (20) en apportant de la vapeur dans la chambre de culture (20), dans lequel
un conduit (22) disposé dans la chambre de culture (20) et un ventilateur (23) pour la circulation de la vapeur sont disposés dans le boîtier (10), la vapeur étant mise en circulation entre le conduit (22) et la chambre de culture (20), et
le capteur d'humidité (27a) est disposé à l'extérieur du conduit (22) dans la chambre de culture (20).

6. Appareil de culture (1) selon la revendication 5, dans lequel
le capteur d'humidité (27a) est disposé dans une position adjacente à une sortie de vapeur dans le conduit (22).

7. Appareil de culture (1) selon la revendication 6, dans lequel :
la chambre de culture (20) est configurée de telle sorte qu'une étagère (26) sur laquelle est placée une culture puisse être installée à une position prédéterminée dans une direction verticale ; et
le capteur d'humidité (27a) est disposé en dessous de la position la plus basse à laquelle l'étagère peut être installée.
